# EUROPÄISCHE PATENTSCHRIFT

(11) **EP 0 601 353 B1**
(45) Veröffentlichungstag und Bekanntmachung des Hinweises auf die Patenterteilung: **23.07.1997**
(21) Anmeldenummer: 93118382.6
(22) Anmeldetag: 12.11.1993
(51) Int. Cl.: C07D 295/088

(54) **Verfahren zur Herstellung von N-(2-Sulfatoethyl)piperazin hoher Reinheit**
Process for the production of N-(2-Sulphatoethyl) piperazine with a high purity
Procédé pour la préparation de N-(2-sulphatoethyle)pipérazine de pureté elevée

(30) Priorität: 21.11.1992 DE 4239183
(43) Veröffentlichungstag der Anmeldung: 15.06.1994
(73) Patentinhaber: HOECHST AKTIENGESELLSCHAFT, 65929 Frankfurt am Main (DE)
(72) Erfinder: Meier, Michael, Dr., D-65835 Liederbach (DE); Kautz, Heinz-Georg, D-63633 Birstein (DE)

(56) Entgegenhaltungen:
- ANDREW STREITWEISER JR. 'Introduction to Organic Chemistry. Seite 815' 1981 , MACMILLAN PUBLISHING CO., INC. , U.S.A.
- BARTON & OLLIS 'comprehensive organic chemistry. Seite 370, Reaktion (7).' 1979 , PERGAMON PRESS , U.K.
- JOURNAL OF HETEROCYCLIC CHEMISTRY Bd. IX , 1972 Seiten 891 - 894 TOMALIA 'The Synthesis and Reactions of beta-substituted Ethyl Sulfates.'

## Beschreibung

Die Erfindung betrifft ein Verfahren zur Herstellung von N-(2-Sulfatoethyl)piperazin hoher Reinheit durch Umsetzen von N-(2-Hydroxyethyl)piperazin in einem Gemisch aus hoch oder höher konzentrierter Schwefelsäure und Oleum oder Chlorsulfonsäure, Eintragen des erhaltenen Sulfiergemisches in einen mit Wasser mischbaren aliphatischen Alkohol, Isolierung des gebildeten N-(2-Sulfatoethyl)piperazin-sulfats, Umsetzung des N-(2-Sulfatoethyl)piperazin-sulfats mit einer basischen Verbindung in einem Gemisch aus einem Alkanol(C₁-C₂) und Wasser und Abtrennung des ausgefallenen Sulfats der eingesetzten basischen Verbindung.

Das N-(2-Sulfatoethyl)piperazin kann als Mittel zur Vorbehandlung und Modifikation von Fasermaterialien, wie synthetischen Polyamid- oder Polyurethanfasermaterialien, Wolle, Seide oder Cellulosefasermaterialien, für das anschließende Färben mit anionischen Farbstoffen eingesetzt werden (Patentanmeldung P 41 40 410.6 und P 42 24 283.5).

In J. Heterocycl. Chem 9, 891-894 wird beschrieben, N-(2-Sulfatoethyl)piperazin könne durch Umsetzung von Piperazin mit Ethylensulfat in Methylenchlorid in 70 %iger Ausbeute als viskoses Öl erhalten werden. Bei der Nacharbeitung dieses Verfahrens ist es uns nicht gelungen, N-(2-Sulfatoethyl)piperazin herzustellen. Es konnte nur ein Produktgemisch erhalten werden (vgl. hierzu das weiter unten angegebene Beispiel 4). Davon abgesehen, ist bei dem beschriebenen Verfahren nachteilig, daß das eingesetzte Ethylensulfat technisch nicht verfügbar ist.

Es bestand daher ein Bedürfnis nach einem wirtschaftlichen und technisch leicht durchführbaren Verfahren zur Herstellung von N-(2-Sulfatoethyl)piperazin in hoher Reinheit.

Es wurde nun überraschenderweise gefunden, daß man N-(2-Sulfatoethyl)piperazin in vorteilhafter Weise und in hoher Reinheit herstellen kann, indem man N-(2-Hydroxyethyl)piperazin in
a) 100 %iger Schwefelsäure
b) einem Gemisch aus 100 %iger Schwefelsäure und Oleum oder Chlorsulfonsäure oder
c) einem Gemisch aus Schwefelsäure mit einem Gehalt an H₂SO₄ von weniger als 100 % und Oleum oder Chlorsulfonsäure bei Temperaturen von etwa 35 bis etwa 90°C, vorzugsweise etwa 40 bis etwa 70°C, mit einer basischen Verbindung in einem Gemisch aus einem Alkanol (C₁-C₂) und Wasser behandelt, das ausgefallene Sulfat der eingesetzten basischen Verbindung abtrennt und durch Zugabe von Alkanol (C₁-C₂) das gebildete N-(2-Sulfatoethyl)piperazin isoliert.

Beim erfindungsgemäßen Verfahren setzt man zweckmäßigerweise 100 %ige Schwefelsäure in etwa 0,8 bis etwa 3, bevorzugt etwa 1,3 bis etwa 1,7 Gewichtsteilen je Gewichtsteil N-(2-Hydroxyethyl)piperazin ein. Es können auch größere Mengen an Schwefelsäure verwendet werden. Dies ergibt jedoch keine Qualitätsvorteile und es müssen lediglich größere Mengen Schwefelsäure entsorgt werden. Anstelle 100 %iger Schwefelsäure kann auch eine Schwefelsäure mit niedrigerer Konzentration, z.B. 95 bis 99 %ige Schwefelsäure, wie 96 %ige Schwefelsäure, eingesetzt werden. In diesen Fällen muß, entsprechend der zusätzlich anwesenden Wassermenge, mehr Schwefeltrioxid (im Oleum) bzw. Chlorsulfonsäure verwendet werden.

Bei der Verwendung einer Mischung aus 100 %iger Schwefelsäure und Oleum (Lösung von Schwefeltrioxid in 100 %iger Schwefelsäure) wird Schwefeltrioxid in etwa 0,3 bis etwa 0,8, bevorzugt etwa 0,4 bis etwa 0,7, besonders bevorzugt etwa 0,55 bis etwa 0,65 Gewichtsteilen je Gewichtsteil N-(2-Hydroxyethyl)piperazin eingesetzt. Bei der Verwendung einer Mischung aus höher konzentrierter Schwefelsäure und Chlorsulfonsäure wird die Chlorsulfonsäure in etwa 0,6 bis etwa 1,3, bevorzugt etwa 0,7 bis etwa 1,0, besonders bevorzugt etwa 0,80 bis etwa 0,95 Gewichtsteilen je Gewichtsteil N-(2-(Hydroxyethyl)piperazin eingesetzt.

Als Alkanole (C₁-C₂) werden Methanol und/oder Ethanol eingesetzt.

Die Menge des bevorzugt verwendeten wäßrigen Methanols bzw. Ethanols für die Fällung des N-(2-Sulfatoethyl)piperazin-sulfats beträgt etwa 2,0 bis etwa 6,0, bevorzugt etwa 3,0 bis etwa 5,0, besonders bevorzugt etwa 3,2 bis etwa 3,8 Gewichtsteile je Gewichtsteil N-(2-Hydroxyethyl)piperazin. Der Wassergehalt des Alkohols sollte zwischen etwa 10 und etwa 30, bevorzugt zwischen etwa 15 bis etwa 25 Gew.-% betragen. Die Fällung wird bei einer Temperatur von etwa 45°C begonnen und dann durch Abkühlen auf etwa 15 bis etwa 25°C zur vollständigen Kristallisation beendet. Zur Kristallisation werden etwa 0,005 bis etwa 10, bevorzugt etwa 0,01 bis etwa 5 und besonders bevorzugt etwa 0,05 bis etwa 1 Gewichtsteile N-(2-Sulfatoethyl)piperazin-sulfat je 130 Gewichtsteile N-(2-Hydroxyethyl)piperazin zum Fällungsgemisch gegeben. Es können aber auch größere Mengen Impfkristalle zugesetzt werden.

Für die weitere Umsetzung (Überführung des N-(2-Sulfatoethyl)piperazin-sulfats in das hochreine N-(2-Sulfatoethyl)piperazin) kann das N-(2-Sulfatoethyl)piperazin-sulfat noch alkoholfeucht, d.h. ohne Zwischentrocknung, eingesetzt werden.

Hierbei ist es zweckmäßig, das beispielsweise noch methanolfeuchte N-(2-Sulfatoethyl)piperazin-sulfat in ein Gemisch aus etwa 0,7 bis etwa 1 Gewichtsteilen Methanol pro Gewichtsteil N-(2-Sulfatoethyl)piperazin-sulfat, abzüglich der Gewichtsteile Methanol, die sich noch am methanolfeuchten N-(2-Sulfatoethyl)piperazin-sulfat befinden, und etwa 0,5 bis etwa 0,7 Gewichtsteilen Wasser einschließlich des Wassers, das gegebenenfalls mit der wäßrigen Lösung der alkalischen Verbindung in das Reaktionsgemisch eingebracht wird, oder das beispielsweise noch ethanolfeuchte N-(2-Sulfatoethyl)piperazin-sulfat in ein Gemisch aus etwa 1,0 bis etwa 1,3 Gewichtsteilen Ethanol pro Gewichtsteil N-(2-Sulfatoethyl)piperazin-sulfat, abzüglich der Gewichtsteile Ethanol, die sich noch am ethanolfeuchten N-(2-Sulfatoethyl)piperazin-sulfat befinden, und etwa 0,4 bis etwa 0,7 Gewichtsteilen Wasser einschließlich des Wassers, das gegebenenfalls mit der wäßrigen Lösung der alkalischen Verbindung in das Reaktionsgemisch eingebracht wird, einzutragen.

Als basische Verbindung kann beispielsweise ein Alkalimetallhydroxid, - hydrogencarbonat, -carbonat, -methanolat und/oder -ethanolat oder eine Substanz, die im Reaktionsmedium eine dieser Verbindungen bildet, verwendet werden. Bevorzugt werden Natrium- oder Kaliumhydroxid, -hydrogencarbonat, - carbonat, -methanolat und/oder -ethanolat, besonders bevorzugt die Natriumverbindungen, wie beispielsweise Natriumhydroxid, eingesetzt. Die basischen Verbindungen können als solche oder in Form wäßriger Lösungen eingesetzt werden. Da als basische Verbindungen besonders bevorzugt die Natriumverbindungen eingesetzt werden, fällt beim erfindungsgemäßen Verfahren vorwiegend Natriumsulfat aus, das abgetrennt wird.

Die Base wird zweckmäßigerweise in der etwa doppeltstöchiometrischen Menge, bezogen auf das N-(2-Sulfatoethyl)piperazin-sulfat, eingesetzt, und zwar so, daß ein pH-Wert von etwa 6,0 bis etwa 7,8, bevorzugt etwa 6,2 bis etwa 7,5, besonders bevorzugt etwa 6,7 bis etwa 7,1 eingestellt wird.

Zur Isolation des N-(2-Sulfatoethyl)piperazins wird nach dem Abtrennen des Sulfats der eingesetzten Base, beispielsweise des Natriumsulfats, das Filtrat mit etwa 1,4 bis etwa 2,6 Gewichtsteilen Alkohol je Gewichtsteil eingesetztem N-(2-Sulfatoethyl)piperazin-sulfat, versetzt.

Auf diese Weise erhält man N-(2-Sulfatoethyl)piperazin in Ausbeuten von ≥84 % mit Reinheiten ≥95 %. N-(2-Sulfatoethyl)piperazin fällt, im Gegensatz zum Stand der Technik (siehe weiter oben) (viskoses Öl), als kristallines Pulver mit einem Zersetzungspunkt von 232°C an.

Der bei der Sulfierung (Veresterung) angefallene schwefelsäurehaltige Alkohol kann durch Redestillation im Vakuum wiedergewonnen und wiederverwendet werden. Die alkoholischen Mutterlaugen aus der Freisetzung des N-(2-Sulfatoethyl)piperazins aus dessen Salz können ohne weitere Reinigung wieder verwendet werden. Hierdurch können die Abfallmengen deutlich reduziert werden.

Es ist als überraschend zu erachten, daß bei den erfindungsgemäß angewandten Temperaturen und Alkohol/Wasser-Gemischen eine einfache und saubere Trennung von N-(2-Sulfatoethyl)piperazin und Alkalimetallsulfat, vorzugsweise Natriumsulfat, möglich ist.

Die nachstehenden Beispiele dienen zur Erläuterung des erfindungsgemäßen Verfahrens, ohne es darauf zu beschränken.

### Beispiel 1

In einem 1 l-Vierhalskolben mit Rührer, Tropftrichter, Thermometer und Rückflußkühler werden 400,0 g Schwefelsäure (100 %ig) vorgelegt und 260,4 g (2,0 mol) N-(2-Hydroxyethyl)piperazin ca. 20 Minuten lang so zugegeben, daß die Temperatur nicht über 150°C steigt. Anschließend dosiert man 246,2 g Oleum 65 %ig ebenfalls so zu, daß 150°C nicht überschritten werden. Das Reaktionsgemisch (906 g) wird in einen Tropftrichter überführt und dann davon ca. 1/3 in 890 g Ethanol (80 %ig) so eindosiert, daß die Temperatur 45°C nicht überschritten wird. Dann kühlt man unter Rühren auf ca. 20°C ab, impft gegebenenfalls mit 0,1 g N-(2-Sulfatoethyl)piperazin-sulfat und rührt heftig bis das Produkt kristallin ausfällt. Danach läßt man das restliche Reaktionsgemisch in 10 Minuten so zulaufen, daß die Temperatur 30°C nicht übersteigt. Anschließend wird unter Rühren auf 20°C abgekühlt und abgesaugt. Das Produkt wird mit 900 g Ethanol (96 %ig) nachgewaschen. Man erhält 848,2 g N-(2-Sulfatoethyl)piperazin-sulfat ethanolfeucht.

In einem 2 l-Vierhalskolben mit Rührer und Innenthermometer werden 339 g Wasser und 470,8 g Ethanol (96 %ig) vorgelegt und 80,0 g (4,0 mol) Natriumhydroxid eingetragen. Nachdem die Lösung auf 25°C abgekühlt wurde, werden 848,2 g N-(2-Sulfatoethyl)piperazin-sulfat ethanolfeucht eingetragen. Die Suspension wird auf 65°C erwärmt und 30 Minuten bei dieser Temperatur nachgerührt. Der pH-Wert muß 6,7 bis 7,1 betragen und wird eventuell mit Natronlauge bzw. Schwefelsäure eingestellt. Dann wird das ausgefallene Natriumsulfat abgesaugt. Der Filterkuchen wird mit 314 g Ethanol (96 %ig), das auf 65°C erwärmt ist, gewaschen. Man erhält 320,4 g Natriumsulfat ethanolfeucht. Das Filtrat wird mit 813 g Ethanol und der Waschlauge versetzt und das ausgefallene Produkt abgesaugt und mit 300 g Ethanol (96 %ig) gewaschen. Man erhält nach dem Trocknen des Filterkuchens bei 70°C und 100 mbar 380,3 g N-(2-Sulfatoethyl)piperazin als weißes Pulver. Der Reingehalt nach Titration beträgt 95,5 %, was 363,2 g (1,73 mol) N-(2-Sulfatoethyl)piperazin (100 %ig gerechnet) und einer Ausbeute von 86,4 % d.Th. entspricht.
Schmp.: 232°C (Zersetzung, abhängig von der Heizrate)

| | | | | |
|---|---|---|---|---|
| Berechnet für C₆H₁₄N₂O₄S: | C 34,3 % | H 6,6 % | N 13,3 % | S 15,2 % |
| Gefunden: | C 33,2 % | H 6,5 % | N 12,9 % | S 14,9 % |

¹H-NMR (D₆ DMSO): δ = 2,58 - 2,67 (m; 6H; N(CH₂)₃), 3,04 - 3,11 (m; 4H; HN(CH₂)₂), 3,84 (t; 2H; CH₂-O), 8,2 - 8,6 (breit; 2H; NH, OSO₃H).

### Beispiel 2

In einem 1 l-Vierhalskolben mit Rührer, Tropftrichter, Thermometer, Rückflußkühler und Gaseinleitung werden 206,3 g Schwefelsäure (96 %ig) vorgelegt und 130,2 g (1,0 mol) N-(2-Hydroxyethyl)piperazin ca. 20 Minuten lang unter Kühlung so zugegeben, daß die Temperatur nicht über 150°C steigt. Dann werden bei 120°C unter Erwärmen 169,9 g Chlorsulfonsäure 1 Stunde lang zudosiert. Hierbei wird ständig intensiv Stickstoff eingeleitet, weil das Reaktionsgemisch schäumt (HCl-Entwicklung). Nach beendeter Zugabe wird unter Stickstoffeinleitung bei 150°C nachgerührt bis kein Chlorwasserstoff im Abgas nachweisbar ist (ca. 1,5 Stunden). Das Raktionsgemisch (446,9 g) wird in einen Tropftrichter überführt und dann davon ca. 1/3 in 447 g Ethanol (80 %ig) so eindosiert, daß die Temperatur 45°C nicht übersteigt. Anschließend wird, wie in Beispiel 1 beschrieben, weiter umgesetzt. Nach dem Trocknen erhält man bei 70°C und 100 mbar 189,2 g N-(2-Sulfatoethyl)piperazin als weißes Pulver. Der Reingehalt nach Titration beträgt 95,0 %, was 179,7 g (0,86 mol) N-(2-Sulfatoethyl)piperazin (100 %ig gerechnet) und einer Ausbeute von 85,6 % d.Th. entspricht.
- Schmp.:: 237°C (Zersetzung, abhängig von der Heizrate)
Die spektroskopischen und analytischen Daten sind mit den in Beispiel 1 angegebenen identisch.

### Beispiel 3

In einem 1 l-Vierhalskolben mit Rührer, Tropftrichter, Thermometer und Rückflußkühler werden 200 g Schwefelsäure (100 %ig) vorgelegt und 130,2 g (1,0 mol) N-(2-Hydroxyethyl)-piperazin ca. 20 Minuten lang so zugegeben, daß die Temperatur nicht über 200°C steigt. Dann werden 123,1 g Oleum 1 Stunde lang so zudosiert, daß die Temperatur 200°C nicht überschreitet. Darauf wird das Reaktionsgemisch (453,2 g) in einen Tropftrichter überführt und dann davon ca. 1/3 in 453 g Methanol (80 %ig) so eindosiert, daß die Temperatur 45°C nicht übersteigt. Anschließend kühlt man unter Rühren auf ca. 25°C ab, impft gegebenenfalls mit 0,1 g N-(2-Sulfatoethyl)piperazin-sulfat-Kristallen und rührt heftig bis das Produkt kristallin ausfällt. Danach läßt man das restliche Reaktionsgemisch in 10 Minuten so zulaufen, daß die Temperatur 25°C nicht übersteigt. Anschließend wird noch 30 Minuten bei 25°C nachgerührt und abgesaugt. Das Produkt wird mit 412 g Methanol gewaschen. Man erhält 394,2 g N-(2-Sulfatoethyl)piperazin-sulfat methanolfeucht.

In einem 1 l-Vierhalskolben mit Rührer und Innenthermometer werden 186 g Wasser vorgelegt und 80,0 g (2,0 mol) Natriumhydroxid eingetragen. Anschließend werden 172,3 g Methanol eindosiert. In diese Lösung werden 394,2 g N-(2-Sulfatoethyl)piperazin-sulfat methanolfeucht eingetragen. Die Suspension wird auf 45°C erwärmt und 30 Minuten bei dieser Temperatur nachgerührt. Der pH-Wert der Suspension muß 6,7 bis 7,1 betragen. Das ausgefallene Natriumsulfat wird abgesaugt. Der Filterkuchen wird mit 200 g Methanol, das auf 45°C erwärmt ist, gewaschen. Man erhält 135,8 g Natriumsulfat methanolfeucht. Das Filtrat (Mutterlauge und Waschmethanol) wird mit 370 g Methanol versetzt, auf <5°C abgekühlt, 30 Minuten bei dieser Temperatur nachgerührt und das ausgefallene Produkt abgesaugt. Der Filterkuchen wird mit 150 g Methanol gewaschen. Man erhält nach dem Trocknen des Filterkuchens bei 70°C und 100 mbar 183,0 g N-(2-Sulfatoethyl)piperazin als weißes Pulver. Der Reingehalt nach Titration beträgt 97,0 %, was 177,5 g (0,84 mol) N-(2-Sulfatoethyl)piperazin (100 %ig gerechnet) und einer Ausbeute von 84,4 % d.Th. entspricht.
- Schmp.:: 237°C (Zersetzung, abhängig von der Heizrate)
Die spektroskopischen und analytischen Daten sind mit den in Beispiel 1 angegebenen identisch.

### Beispiel 4 (Vergleichsbeispiel gemäß Tomalia et al., J. Heterocycl. Chem. 9, 891-894 (1972))

In einem 500 ml-Vierhalskolben mit Rührer, Thermometer und Rückflußkühler werden 22,2 g (0,18 mol) Ethylensulfat (Herstellung siehe Beispiel 5) und 15,4 g (0,18 mol) Piperazin in 135 ml Methylenchlorid 4 Stunden zum Rückfluß erhitzt. Die Reaktionsmischung wurde auf 5°C abgekühlt. Die sich abscheidende zähe klebrige Masse wird abgetrennt. Nach dem Trocknen fallen 26,2 g an. Potentiometrische Titration und ¹H-NMR sind nicht identisch mit N-(2-Sulfatoethyl)piperazin.

### Beispiel 5 (Vergleichsbeispiel nach DE 10 49 870, Beispiel 1)

23,4 ml Schwefelsäure-Monohydrat werden in 30 Minuten bei 23°C mit 46 g Ethylensulfit versetzt. Nach Ende der Gasentwicklung wird noch 30 Minuten nachgerührt und bei Raumtemperatur mit 128 ml Thionylchlorid unter Rühren versetzt. Nach beendeter Gasentwicklung wird 15 Stunden zum Sieden erhitzt. Anschließend wird zuerst das überschüssige Thionylchlorid bei Normaldruck und dann bei 100 mbar abdestilliert. Der Rückstand wird bei 15 mbar destilliert. Es fallen 46,3 g dunkelbraunes Destillat an, das mit Ether extrahiert wird. Es werden 28,9 g eines beigefarbenen Pulvers erhalten (Ausbeute: 54,7 % d.Th.).

## Patentansprüche

1. Verfahren zur Herstellung von N-(2-Sulfatoethyl)piperazin, dadurch gekennzeichnet, daß man N-(2-Hydroxyethyl)piperazin in
a) 100 %iger Schwefelsäure
b) einem Gemisch aus 100 %iger Schwefelsäure und Oleum oder Chlorsulfonsäure oder
c) einem Gemisch aus Schwefelsäure mit einem Gehalt an H₂SO₄ von weniger als 100 % und Oleum oder Chlorsulfonsäure bei Temperaturen von 80 bis 250°C umsetzt, das angefallene Sulfiergemisch in einen mit Wasser mischbaren aliphatischen Alkohol einträgt, das gebildete N-(2-Sulfatoethyl)piperazin-sulfat isoliert, das noch alkoholfeuchte N-(2-Sulfatoethyl)piperazin-sulfat bei Temperaturen von 35 bis 90°C mit einer basischen Verbindung in einem Gemisch aus einem Alkanol (C₁-C₂) und Wasser behandelt, das ausgefallene Sulfat der eingesetzten basischen Verbindung abtrennt und durch Zugabe von Alkanol (C₁-C₂) das gebildete N-(2-Sulfatoethyl)piperazin isoliert.

2. Verfahren nach Anspruch 1, dadurch gekennzeichnet, daß man das (N-(2-Hydroxyethyl)-piperazin bei Temperaturen von 100°C bis 170°C umsetzt.

3. Verfahren nach mindestens einem der Ansprüche 1 und 2, dadurch gekennzeichnet, daß man das noch alkoholfeuchte N-(2-Sulfatoethyl)piperazin-sulfat mit der basischen Verbindung bei Temperaturen von 40 bis 70°C behandelt.

4. Verfahren nach mindestens einem der Ansprüche 1 bis 3, dadurch gekennzeichnet, daß man mit einem Alkalimetallhydroxid, -hydrogencarbonat, -carbonat, -methanolat und/oder -ethanolat oder mit einer diese Verbindungen im Reaktionsmedium bildenden Substanz als basischer Verbindung behandelt.

5. Verfahren nach mindestens einem der Ansprüche 1 bis 4, dadurch gekennzeichnet, daß man Natrium- oder Kaliumhydroxid, -hydrogencarbonat, -carbonat, -methanolat und/oder -ethanolat oder mit einer dieser Verbindungen im Reaktionsmedium bildenden Substanz als basischer Verbindung behandelt.

6. Verfahren nach mindestens einem der Ansprüche 1 bis 5, dadurch gekennzeichnet, daß man als Alkanol (C₁-C₁₂) Methanol und/oder Ethanol verwendet.

7. Verfahren nach mindestens einem der Ansprüche 1 bis 6, dadurch gekennzeichnet, daß die Schwefelsäure mit einem Gehalt an H₂SO₄ von weniger als 100 % einen H₂SO₄-Gehalt von mindestens 95 % aufweist.

8. Verfahren nach mindestens einem der Ansprüche 1 bis 6, dadurch gekennzeichnet, daß man in 0,8 bis 3 Gewichtsteilen 100 %iger Schwefelsäure je Gewichtsteil N-(2-Hydroxyethyl)piperazin umsetzt.

9. Verfahren nach mindestens einem der Ansprüche 1 bis 6, dadurch gekennzeichnet, daß man in 1,3 bis 1,7 Gewichtsteilen 100 %iger Schwefelsäure je Gewichtsteil N-(2-Hydroxyethyl)piperazin umsetzt.

10. Verfahren nach mindestens einem der Ansprüche 1 bis 6, dadurch gekennzeichnet, daß bei der Umsetzung in einer Mischung aus 100 %iger Schwefelsäure und Oleum der Gehalt des Oleums an Schwefeltrioxid 0,3 bis 0,8 Gewichtsteilen je Gewichtsteil (N-(2-Hydroxyethyl)piperazin beträgt.

11. Verfahren nach mindestens einem der Ansprüche 1 bis 6, dadurch gekennzeichnet, daß bei der Umsetzung in einer Mischung aus 100 %iger Schwefelsäure und Oleum der Gehalt des Oleums an Schwefeltrioxid 0,4 bis 0,7 Gewichtsteile je Gewichtsteil N-(2-Hydroxyethyl)piperazin beträgt.

12. Verfahren nach mindestens einem der Ansprüche 1 bis 6, dadurch gekennzeichnet, daß bei der Umsetzung in einer Mischung aus 100 %iger Schwefelsäure und Oleum der Gehalt des Oleums an Schwefeltrioxid 0,55 bis 0,65 Gewichtsteile je Gewichtsteil N-(2-Hydroxyethyl)piperazin beträgt.

13. Verfahren nach mindestens einem der Ansprüche 1 bis 6, dadurch gekennzeichnet, daß man bei der Umsetzung in einem Gemisch aus 100 %iger Schwefelsäure und Chlorsulfonsäure 0,6 bis 1,3 Gewichtsteile Chlorsulfonsäure je Gewichtsteil N-(2-Hydroxyethyl)piperazin einsetzt.

14. Verfahren nach mindestens einem der Ansprüche 1 bis 6, dadurch gekennzeichnet, daß man bei der Umsetzung in einem Gemisch aus 100 %iger Schwefelsäure und Chlorsulfonsäure 0,7 bis 1,0 Gewichtsteile Chlorsulfonsäure je Gewichtsteil N-(2-Hydroxyethyl)piperazin einsetzt.

15. Verfahren nach mindestens einem der Ansprüche 1 bis 6, dadurch gekennzeichnet, daß man bei der Umsetzung in einem Gemisch aus 100 %iger Schwefelsäure und Chlorsulfonsäure 0,80 bis 0,95 Gewichtsteile Chlorsulfonsäure je Gewichtsteil N-(2-Hydroxyethyl)piperazin einsetzt.

16. Verfahren nach mindestens einem der Ansprüche 1 bis 15, dadurch gekennzeichnet, daß man das angefallene Sulfiergemisch in 2,0 bis etwa 6,0 Gewichtsteile Methanol oder Ethanol je Gewichtsteil N-(2-Hydroxyethyl)piperazin einträgt.

17. Verfahren nach mindestens einem der Ansprüche 1 bis 16, dadurch gekennzeichnet, daß man das angefallene Sulfiergemisch in etwa 3,0 bis etwa 5,0 Gewichtsteile Methanol oder Ethanol je Gewichtsteil N-(2-Hydroxyethyl)piperazin einträgt.

18. Verfahren nach mindestens einem der Ansprüche 1 bis 16, dadurch gekennzeichnet, daß man das angefallene Sulfiergemisch in etwa 3,2 bis etwa 3,8 Gewichtsteile Methanol oder Ethanol je Gewichtsteil N-(2-Hydroxyethyl)piperazin einträgt.

19. Verfahren nach mindestens einem der Ansprüche 1 bis 18, dadurch gekennzeichnet, daß man das Sulfiergemisch in Methanol oder Ethanol mit einem Wassergehalt von etwa 10 bis 30 Gew.-% einträgt.

20. Verfahren nach mindestens einem der Ansprüche 1 bis 18, dadurch gekennzeichnet, daß man das Sulfiergemisch in Methanol oder Ethanol mit einem Wassergehalt von etwa 15 bis etwas 25 Gew.-% einträgt.

21. Verfahren nach mindestens einem der Ansprüche 1 bis 20, dadurch gekennzeichnet, daß man dem Sulfiergemisch Impfkristalle aus N-(2-Sulfatoethyl)piperazin-sulfat zusetzt.

22. Verfahren nach mindestens einem der Ansprüche 1 bis 21, dadurch gekennzeichnet, daß man den nach der Isolation des gebildeten N-(2-Sulfatoethyl)piperazin-sulfats angefallenen schwefelsäurehaltigen Alkohol durch Destillation wiedergewinnt und in einem Folgeansatz verwendet.

23. Verfahren nach mindestens einem der Ansprüche 1 bis 22, dadurch gekennzeichnet, daß man das noch methanolfeuchte N-(2-Sulfatoethyl)piperazin-sulfat in ein Gemisch aus etwa 0,7 bis etwa 1 Gewichtsteilen Methanol pro Gewichtsteil N-(2-Sulfatoethyl)piperazin-sulfat, abzüglich der Gewichtsteile Methanol, die sich noch am methanolfeuchten N-(2-Sulfatoethyl)piperazin-sulfat befinden, und etwa 0,5 bis etwa 0,7 Gewichtsteilen Wasser einschließlich des Wassers, das gegebenenfalls mit der wäßrigen Lösung der basischen Verbindung in das Reaktionsgemisch eingebracht wird, einträgt.

24. Verfahren nach mindestens einem der Ansprüche 1 bis 22, dadurch gekennzeichnet, daß man das ethanolfeuchte N-(2-Sulfatoethyl)piperazin-sulfat in ein Gemisch aus etwa 1,0 bis etwa 1,3 Gewichtsteilen Ethanol pro Gewichtsanteil N-(2-Sulfatoethyl)piperazin-sulfat, abzüglich der Gewichtsteile Ethanol, die sich noch am ethanolfeuchten N-(2-Sulfatoethyl)piperazin-sulfat befinden, und etwa 0,4 bis etwa 0,7 Gewichtsteilen Wasser einschließlich des Wassers, das gegebenenfalls mit der wäßrigen Lösung der basischen Verbindung in das Reaktionsgemisch eingebracht wird, einträgt.

25. Verfahren nach mindestens einem der Ansprüche 1 bis 24, dadurch gekennzeichnet, daß man nach Abtrennen des Sulfats der eingesetzten alkalischen Verbindung das Filtrat mit etwa 1,4 bis etwa 2,6 Gewichtsteilen Methanol und/oder Ethanol je Gewichtsteil eingesetztem N-(2-Sulfatoethyl)piperazin-sulfat versetzt.

26. Verfahren nach mindestens einem der Ansprüche 1 bis 25, dadurch gekennzeichnet, daß man die bei der Freisetzung des N-(2-Sulfatoethyl)piperazins mittels der basischen Verbindung angefallenen alkoholischen Mutterlaugen ohne weitere Reinigung wieder verwendet.

## Claims

1. A process for the preparation of N-(2-sulfatoethyl)piperazine, which comprises reacting N-(2-hydroxyethyl)piperazine in
a) 100 % strength sulfuric acid
b) a mixture of 100 % strength sulfuric acid and oleum or chlorosulfonic acid or
c) a mixture of sulfuric acid having a content of H₂SO₄ of less than 100 % and oleum or chlorosulfonic acid at temperatures of from 80 to 250°C, transferring the resulting sulfonation mixture to a water-miscible aliphatic alcohol, isolating the N-(2-sulfatoethyl)piperazine sulfate formed, treating the N-(2-sulfatoethyl)piperazine sulfate, still moist with alcohol, at temperatures of from 35 to 90°C with a basic compound in a mixture of a (C₁-C₂)-alkanol and water, separating off the precipitated sulfate of the basic compound employed, and isolating the N-(2-sulfatoethyl)piperazine formed, by the addition of (C₁-C₂)-alkanol.

2. The process as claimed in claim 1, wherein the N-(2-hydroxyethyl)piperazine is reacted at temperatures of from 100°C to 170°C.

3. The process as claimed in at least one of claims 1 and 2, wherein the N-(2-sulfatoethyl)piperazine sulfate, still moist with alcohol, is treated with the basic compound at temperatures of from 40 to 70°C.

4. The process as claimed in at least one of claims 1 to 3, wherein the treatment with a basic compound is carried out using an alkali metal hydroxide, hydrogen carbonate, carbonate, methanolate and/or ethanolate or using a substance which forms these compounds in the reaction medium.

5. The process as claimed in at least one of claims 1 to 4, wherein the treatment with a basic compound is carried out using the hydroxide, hydrogen carbonate, carbonate, methanolate and/or ethanolate of sodium or of potassium, or using a substance which forms these compounds in the reaction medium.

6. The process as claimed in at least one of claims 1 to 5, wherein the (C₁-C₂)-alkanol used is methanol and/or ethanol.

7. The process as claimed in at least one of claims 1 to 6, wherein the sulfuric acid having a content of H₂SO₄ of less than 100 % has an H₂SO₄ content of at least 95 %.

8. The process as claimed in at least one of claims 1 to 6, wherein the reaction is carried out in from 0.8 to 3 parts by weight of 100 % strength sulfuric acid per part by weight of N-(2-hydroxyethyl)piperazine.

9. The process as claimed in at least one of claims 1 to 6, wherein the reaction is carried out in from 1.3 to 1.7 parts by weight of 100 % strength sulfuric acid per part by weight of N-(2-hydroxyethyl)piperazine.

10. The process as claimed in at least one of claims 1 to 6, wherein, when the reaction is carried out in a mixture of 100 % strength sulfuric acid and oleum, the content of sulfur trioxide in the oleum is from 0.3 to 0.8 part by weight per part by weight of N-(2-hydroxyethyl)piperazine.

11. The process as claimed in at least one of claims 1 to 6, wherein, when the reaction is carried out in a mixture of 100 % strength sulfuric acid and oleum, the content of sulfur trioxide in the oleum is from 0.4 to 0.7 part by weight per part by weight of N-(2-hydroxyethyl)piperazine.

12. The process as claimed in at least one of claims 1 to 6, wherein, when the reaction is carried out in a mixture of 100 % strength sulfuric acid and oleum, the content of sulfur trioxide in the oleum is from 0.55 to 0.65 part by weight per part by weight of N-(2-hydroxyethyl)piperazine.

13. The process as claimed in at least one of claims 1 to 6, wherein, when the reaction is carried out in a mixture of 100 % strength sulfuric acid and chlorosulfonic acid, from 0.6 to 1.3 parts by weight of chlorosulfonic acid are employed per part by weight of N-(2-hydroxyethyl)piperazine.

14. The process as claimed in at least one of claims 1 to 6, wherein, when the reaction is carried out in a mixture of 100 % strength sulfuric acid and chlorosulfonic acid, from 0.7 to 1.0 part by weight of chlorosulfonic acid is employed per part by weight of N-(2-hydroxyethyl)piperazine.

15. The process as claimed in at least one of claims 1 to 6, wherein, when the reaction is carried out in a mixture of 100 % strength sulfuric acid and chlorosulfonic acid, from 0.80 to 0.95 part by weight of chlorosulfonic acid is employed per part by weight of N-(2-hydroxyethyl)piperazine.

16. The process as claimed in at least one of claims 1 to 15, wherein the resulting sulfonation mixture is transferred to from 2.0 to about 6.0 parts by weight of methanol or ethanol per part by weight of N-(2-hydroxyethyl)piperazine.

17. The process as claimed in at least one of claims 1 to 16, wherein the resulting sulfonation mixture is transferred to from about 3.0 to about 5.0 parts by weight of methanol or ethanol per part by weight of N-(2-hydroxyethyl)piperazine.

18. The process as claimed in at least one of claims 1 to 16, wherein the resulting sulfonation mixture is transferred to from about 3.2 to about 3.8 parts by weight of methanol or ethanol per part by weight of N-(2-hydroxyethyl)piperazine.

19. The process as claimed in at least one of claims 1 to 18, wherein the sulfonation mixture is transferred to methanol or ethanol having a water content of from about 10 to 30 % by weight.

20. The process as claimed in at least one of claims 1 to 18, wherein the sulfonation mixture is transferred to methanol or ethanol having a water content of from about 15 to about 25 % by weight.

21. The process as claimed in at least one of claims 1 to 20, wherein seed crystals of N-(2-sulfatoethyl)piperazine sulfate are added to the sulfonation mixture.

22. The process as claimed in at least one of claims 1 to 21, wherein the sulfuric acid-containing alcohol obtained after isolating the N-(2-sulfatoethyl)piperazine sulfate formed is recovered by distillation and used in a subsequent batch.

23. The process as claimed in at least one of claims 1 to 22, wherein the N-(2-sulfatoethyl)piperazine sulfate, still moist with methanol, is transferred into a mixture of from about 0.7 to about 1 part by weight of methanol per part by weight of N-(2-sulfatoethyl)piperazine sulfate, deducting the parts by weight of methanol which are still in the methanol-moist N-(2-sulfatoethyl)piperazine sulfate, and from about 0.5 to about 0.7 part by weight of water, including the water possibly introduced into the reaction mixture if the basic compound is in aqueous solution.

24. The process as claimed in at least one of claims 1 to 22, wherein the N-(2-sulfatoethyl)piperazine sulfate, still moist with ethanol, is transferred into a mixture of from about 1.0 to about 1.3 parts by weight of ethanol per part by weight of N-(2-sulfatoethyl)piperazine sulfate, deducting the parts by weight of ethanol which are still in the ethanol-moist N-(2-sulfatoethyl)piperazine sulfate, and from about 0.4 to about 0.7 part by weight of water, including the water possibly introduced into the reaction mixture if the basic compound is in aqueous solution.

25. The process as claimed in at least one of claims 1 to 24, wherein from about 1.4 to about 2.6 parts by weight of methanol and/or ethanol per part by weight of N-(2-sulfatoethyl)piperazine sulfate employed are added to the filtrate obtained after separating off the sulfate of the alkaline compound employed.

26. The process as claimed in at least one of claims 1 to 25, wherein the alcoholic mother liquors obtained in the course of liberation of the N-(2-sulfatoethyl)piperazine using the basic compound are used again without further purification.

## Revendications

1. Procédé de préparation de la N-(2-sulfatoéthyl)pipérazine, caractérisé en ce que l'on fait réagir de la N-(2-hydroxyéthyl)pipérazine dans
a) de l'acide sulfurique à 100 %
b) un mélange d'acide sulfurique à 100 % et d'oléum ou d'acide chlorosulfonique, ou
c) un mélange d'acide sulfurique ayant une teneur en H₂SO₄ inférieure à 100 % et d'oléum ou d'acide chlorosulfonique à des températures de 80 à 250 °C, on introduit le mélange de sulfatation obtenu dans un alcool aliphatique miscible à l'eau, on isole le sulfate de N-(2-sulfatoéthyl)pipérazine formé, on traite le sulfate N-(2-sulfatoéthyl)pipérazine encore humidifié par de l'alcool à des températures de 35 à 90 °C au moyen d'un composé basique dans un mélange d'alcool en C₁-C₂ et d'eau, on sépare le sulfate précipité du composé basique utilisé et on isole la N-(2-sulfatoéthyl)pipérazine formée par addition d'alcanol en C₁-C₂.

2. Procédé selon la revendication 1, caractérisé en ce que l'on fait réagir la N-(2-hydroxyéthyl)pipérazine à des températures de 100 °C à 170 °C.

3. Procédé selon au moins une des revendications 1 et 2, caractérisé en ce que l'on traite le sulfate de N-(2-sulfatoéthyl)pipérazine encore humidifié par de l'alcool avec le composé basique à des températures de 40 à 70 °C.

4. Procédé selon au moins une des revendications 1 à 3, caractérisé en ce que l'on traite avec un hydroxyde, un bicarbonate, un carbonate, un méthanolate et/ou un éthanolate de métal alcalin ou avec une substance formant l'un de ces composés dans le mélange réactionnel, en tant que composé basique.

5. Procédé selon au moins une des revendications 1 à 4, caractérisé en ce que l'on traite avec de l'hydroxyde, du bicarbonate, du carbonate, du méthanolate et/ou de l'éthanolate de sodium ou de potassium ou avec une substance formant l'un de ces composés dans le mélange réactionnel, en tant que composé basique.

6. Procédé selon au moins une des revendications 1 à 5, caractérisé en ce que l'on utilise du méthanol et/ou de l'éthanol en tant qu'alcanol en C₁-C₂.

7. Procédé selon au moins une des revendications 1 à 6, caractérisé en ce que l'acide sulfurique ayant une teneur en H₂SO₄ inférieure à 100 % présente une teneur en H₂SO₄ d'au moins 95 %.

8. Procédé selon au moins une des revendications 1 à 6, caractérisé en ce que l'on fait réagir dans 0,8 à 3 parties en poids d'acide sulfurique à 100 % par partie en poids de N-(2-hydroxyéthyl)pipérazine.

9. Procédé selon au moins une des revendications 1 à 6, caractérisé en ce que l'on fait réagir dans 1,3 à 1,7 partie en poids d'acide sulfurique à 100 % par partie en poids de N-(2-hydroxyéthyl)pipérazine.

10. Procédé selon au moins une des revendications 1 à 6, caractérisé en ce que, dans la réaction dans un mélange d'acide sulfurique à 100 % et d'oléum, la teneur de l'oléum en anhydre sulfurique est de 0,3 à 0,8 partie en poids par partie en poids de N-(2-hydroxyéthyl)pipérazine.

11. Procédé selon au moins une des revendications 1 à 6, caractérisé en ce que, dans la réaction dans un mélange d'acide sulfurique à 100 % et d'oléum, la teneur de l'oléum en anhydre sulfurique est de 0,4 à 0,7 partie en poids par partie en poids de N-(2-hydroxyéthyl)pipérazine.

12. Procédé selon au moins une des revendications 1 à 6, caractérisé en ce que, dans la réaction dans un mélange d'acide sulfurique à 100 % et d'oléum, la teneur de l'oléum en anhydre sulfurique est de 0,55 à 0,65 partie en poids par partie en poids de N-(2-hydroxyéthyl)pipérazine.

13. Procédé selon au moins une des revendications 1 à 6, caractérisé en ce que, dans la réaction dans un mélange d'acide sulfurique à 100 % et d'acide chlorosulfonique, on utilise 0,6 à 1,3 partie en poids d'acide chlorosulfonique par partie en poids de N-(2-hydroxyéthyl)pipérazine.

14. Procédé selon au moins une des revendications 1 à 6, caractérisé en ce que, dans la réaction dans un mélange d'acide sulfurique à 100 % et d'acide chlorosulfonique, on utilise 0,7 à 1,0 partie en poids d'acide chlorosulfonique par partie en poids de N-(2-hydroxyéthyl)pipérazine.

15. Procédé selon au moins une des revendications 1 à 6, caractérisé en ce que, dans la réaction dans un mélange d'acide sulfurique à 100 % et d'acide chlorosulfonique, on utilise 0,80 à 0,95 partie en poids d'acide chlorosulfonique par partie en poids de N-(2-hydroxyéthyl)pipérazine.

16. Procédé selon au moins une des revendications 1 à 15, caractérisé en ce que l'on introduit le mélange de sulfatation formé dans environ 2,0 à environ 6,0 parties en poids de méthanol ou d'éthanol par partie en poids de N-(2-hydroxyéthyl)pipérazine.

17. Procédé selon au moins une des revendications 1 à 16, caractérisé en ce que l'on introduit le mélange de sulfatation formé dans environ 0,3 à environ 5,0 parties en poids de méthanol ou d'éthanol par partie en poids de N-(2-hydroxyéthyl)pipérazine.

18. Procédé selon au moins une des revendications 1 à 16, caractérisé en ce que l'on introduit le mélange de sulfatation formé dans environ 3,2 à environ 3,8 parties en poids de méthanol ou d'éthanol par partie en poids de N-(2-hydroxyéthyl)pipérazine.

19. Procédé selon au moins une des revendications 1 à 18, caractérisé en ce que l'on introduit le mélange de sulfatation formé dans du méthanol ou de l'éthanol ayant une teneur en eau d'environ 10 à environ 30 % en poids.

20. Procédé selon au moins une des revendications 1 à 18, caractérisé en ce que l'on introduit le mélange de sulfatation formé dans du méthanol ou de l'éthanol ayant une teneur en eau d'environ 15 à environ 25 % en poids.

21. Procédé selon au moins une des revendications 1 à 20, caractérisé en ce que l'on ajoute des cristaux d'ensemencement en sulfate de N-(2-sulfatoéthyl)pipérazine au mélange de sulfatation.

22. Procédé selon au moins une des revendications 1 à 21, caractérisé en ce que l'on récupère par distillation l'alcool contenant de l'acide sulfurique obtenu après isolement du sulfate de N-(2-sulfatoéthyl)pipérazine formé et en ce qu'on l'utilise dans une charge ultérieure.

23. Procédé selon au moins une des revendications 1 à 22, caractérisé en ce que l'on introduit le sulfate de N-(2-sulfatoéthyl)pipérazine encore humidifié par du méthanol dans un mélange d'environ 0,7 à environ 1 partie en poids de méthanol par partie en poids de sulfate de N-(2-sulfatoéthyl)pipérazine, déduction faite des parties en poids de méthanol qui se trouvent encore sur le sulfate de N-(2-sulfatoéthyl)pipérazine humidifié par du méthanol, et d'environ 0,5 à environ 0,7 partie en poids d'eau, y compris l'eau qui est éventuellement introduite dans le mélange réactionnel avec la solution aqueuse du composé basique.

24. Procédé selon au moins une des revendications 1 à 22, caractérisé en ce que l'on introduit le sulfate de N-(2-sulfatoéthyl)pipérazine humidifié par de l'éthanol dans un mélange d'environ 1,0 à environ 1,3 partie en poids d'éthanol par partie en poids de sulfate de N-(2-sulfatoéthyl)pipérazine, déduction faite des parties en poids d'éthanol qui se trouvent encore sur le sulfate de N-(2-sulfatoéthyl)pipérazine humidifié par de l'éthanol, et d'environ 0,4 à environ 0,7 partie en poids d'eau, y compris l'eau qui est éventuellement introduite dans le mélange réactionnel avec la solution aqueuse du composé alcalin.

25. Procédé selon au moins une des revendications 1 à 24, caractérisé en ce que, après séparation du sulfate du composé alcalin utilisé, on additionne le filtrat avec environ 1,4 à environ 2,6 parties en poids de méthanol et/ou d'éthanol par partie en poids de sulfate de N-(2-sulfatoéthyl)pipérazine utilisé.

26. Procédé selon au moins une des revendications 1 à 25, caractérisé en ce que l'on utilise de nouveau, sans autre purification, les eaux mères alcooliques formées lors de la libération de la N-(2-sulfatoéthyl)pipérazine au moyen du composé basique.
